# EUROPÄISCHE PATENTSCHRIFT

(11) **EP 0 915 839 B1**
(45) Veröffentlichungstag und Bekanntmachung des Hinweises auf die Patenterteilung: **19.09.2001**
(21) Anmeldenummer: 97941891.0
(22) Anmeldetag: 23.07.1997
(51) Int. Cl.: C07C 253/30, C07C 255/24

(54) **VERFAHREN ZUR HERSTELLUNG VON 6-AMINOCAPRONITRIL**
MANUFACTURING PROCESS FOR 6-AMINOCAPRONITRILE
PROCEDE DE PREPARATION DE 6-AMINOCAPRONITRILE

(30) Priorität: 03.08.1996 DE 19631521
(43) Veröffentlichungstag der Anmeldung: 19.05.1999
(73) Patentinhaber: BASF AKTIENGESELLSCHAFT, 67056 Ludwigshafen (DE)
(72) Erfinder: FISCHER, Rolf, D-69121 Heidelberg (DE); PACIELLO, Rocco, D-67098 Bad Dürkheim (DE); RÖPER, Michael, D-67157 Wachenheim (DE); SCHNURR, Werner, D-67273 Herxheim (DE)
(86) Internationale Anmeldenummer: EP9703988
(87) Internationale Veröffentlichungsnummer: WO9805632

(56) Entgegenhaltungen:
- EP-A- 0 011 401
- EP-A- 0 376 121
- WO-A-93/16034
- WO-A-95/18783

## Beschreibung

Die vorliegende Anmeldung betrifft ein Verfahren zur Herstellung von 6-Aminocapronitril oder 6-Aminocapronitril-Hexamethylendiamin-Gemischen.

In der EP-A 11401 wird die Umsetzung von 3-Pentennitril mit Kohlenmonoxid und Wasserstoff unter Druck in Gegenwart eines Cobalt-Katalysators beschrieben, wobei ein Gemisch aus isomeren Formylvaleronitrilen und die entsprechenden aus den Aldehyden gebildeten Alkohole erhalten werden. Ferner wird die reduktive Aminierung von δ-Cyanovaleraldehyd zu Hexamethylendiamin beschrieben. Gemäß Beispiel 4 der genannten Anmeldung wurde ein Gemisch mit 60 % δ-Cyanovaleraldehyd mit Ammoniak und Wasserstoff bei einer Temperatur von 100°C und einem Wasserstoffdruck von 140 bar in Gegenwart von Raney-Nickel während zwei Stunden umgesetzt, wobei der Umsatz (bezogen auf die 8-Verbindung) nur 25 % betrug. Der niedrige Umsetzungsgrad zeigt, daß es sich bei der aminierenden Hydrierung einer Aldehydgruppe und der Hydrierung einer Nitrilgruppe im gleichen Molekül zu einem Diamin um ein schwieriges Hydrierproblem handelt. Ferner wird die Bildung von 6-Aminocapronitril nicht beschrieben. Des weiteren ist die Standzeit des verwendeten Katalysators für eine wirtschaftliche Nutzung unbefriedigend.

Aus US-A 2,777,873 ist bekannt, 5-Formylvaleriansäureester mit Ammoniak und Wasserstoff in Gegenwart von Nickel-, Cobalt-, Eisen-, Platin- oder Palladiumkatalysatoren bei 100 bis 160 °C und Drücken von 1 bis 1 000 Atmosphären aminierend zu 6-Aminocapronsäureestern zu hydrieren. In der EP-A 376 121 ist diese Reaktion auch für Rutheniumkatalysatoren beschrieben, wobei man bei Temperaturen im Bereich von 80 bis 140 °C und Drücken im Bereich von 40 bis 1000 bar arbeitet.

Für die Hydrierung von Adipodinitril zu Hexamethylendiamin in Gegenwart von Ammoniak sind nach US-A 3,461,167, Spalte 3, Zeilen 66 bis 74, Cobalt-, Kupfer- und Rheniumkatalysatoren geeignet. Gearbeitet wird bevorzugt bei 70 bis 170 °C und 300 bis 7 000 p.s.i. Nach der US-A 3,471,563 können für diese Umsetzung auch Rutheniumkatalysatoren verwendet werden.

In der EP-A 214 622 wird die Hydroformylierung von 3-Butennitril in Gegenwart von Rhodium/Chelatphosphitkatalysatoren im beschreibenden Teil erwähnt.

In der WO 94/26688 wird ein Verfahren beschrieben, bei dem man
(a) interne substituierte Olefine zu endständigen Olefinen isomerisiert,
(b) die endständigen Olefine in Gegenwart der internen Olefine bevorzugt hydroformyliert,
(c) die Produkte der Hydroformylierung abgetrennt und
(d) die internen Olefine in die Isomerisierung zurückgeführt werden.

In Anspruch 3 der WO 94/26688 werden nitrilhaltige Olefine beansprucht. Als Hydroformylierungs-Katalysatoren werden Rhodium-Triphenylphosphin-Systeme verwendet, in denen das Triphenylphosphin durch geeignete funktionelle Gruppen wasserlöslich gemacht wird.

In der WO 95/18783 wird die Hydroformylierung von internen nitrilhaltigen Olefinen mit wasserlöslichen Platinkatalysatoren beschrieben.

WO 93/13064 beschreibt ein Verfahren zur Herstellung von 6-Aminoicapronitril durch Hydrierung von Adipodinitril durch Umsetzung einer Mischung aus
a) Adipodinitril,
b) einer Base ausgewählt aus der Gruppe bestehend aus Natrium-, Lithium-, Kalium- und Ammonium-Hydroxid,
c) Wasserstoff,
d) eines Übergangsmetall-Komplexes mit geringer Wertigkeit des Metalls ausgewählt aus der Gruppe bestehend aus Chromhexacarbonyl, Wolframhexacarbonyl, Dibenzolchrom, Dicobaltoctacarbonyl, Chrom-(III)-acetat, Cobalt-(II)-acetat und Eisen-(III)-acetylacetonat und
e) einem Raney-Nickel-Katalysator
bei einem Wasserstoff-Druck von zwischen 1,379 und 6,895 Mpa (200 und 1000 psig) und einer Temperatur von zwischen 50 und 90°C.

Der vorliegenden Erfindung lag daher die Aufgabe zugrunde, ein Verfahren zur Verfügung zu stellen, das es gestattet, ausgehend von 2- und/oder 3- und/oder 4-Pentennitril entweder 6-Aminocapronitril oder ein Gemisch von 6-Aminocapronitril und Hexamethylendiamin mit einem sehr hohen Umsatz herzustellen. Insbesondere war es das Ziel, ein Verfahren zu finden, das lange Verweilzeiten der Katalysatoren garantiert.

Demgemäß wurde ein Verfahren zur Herstellung von 6-Aminocapronitril oder 6-Aminocapronitril/Hexamethylendiamin-Gemischen gefunden, indem man
a) mindestens ein Pentennitril, ausgewählt aus der Gruppe, bestehend aus 2-, 3- und 4-Pentennitril mit Kohlenmonoxid und Wasserstoff in Gegenwart von Katalysatoren umsetzt, die mindestens ein Element der achten Nebengruppe als aktive Komponente enthalten, unter Erhalt eines Hydroformylierungsaustrages I,
b) aus dem Hydroformylierungsaustrag I gewünschtenfalls Kohlenmonoxid, Wasserstoff und den Katalysator abtrennt unter Erhalt eines Hydroformylierungsaustrages II,
c) aus dem Hydroformylierungsaustrag I oder II 5-Formylvaleronitril abtrennt,
d) das abgetrennte 5-Formylvaleronitril mit Ammoniak und Wasserstoff in Gegenwart von Hydrierkatalysatoren, ausgewählt aus der Gruppe, bestehend aus Rhenium, Kupfer und deren Verbindungen sowie Metallen und Metallverbindungen der achten Nebengruppe, umsetzt unter Erhalt eines Hydrieraustrages, und
e) aus dem Hydrieraustrag 6-Aminocapronitril und gegebenenfalls Hexamethylendiamin gewinnt.

Erfindungsgemäß setzt man mindestens ein Pentennitril, ausgewählt aus der Gruppe, bestehend aus 2-, 3- und 4-Pentennitril, bevorzugt 3- oder 4-Pentennitril, ein.

3-Pentennitril, das wichtigste Ausgangsprodukt des Verfahrens, läßt sich beispielsweise durch Anlagerung von Blausäure an Butadien in Gegenwart von Nickel(O)-Phosphit-Komplexen als Katalysatoren herstellen (K. Weissermel, H.-J. Arpe, Industrielle organische Chemie, 4. Auflage, VCH-Verlag Weinheim, 1994, Seite 268).

2- und 4-Pentennitril sind beispielsweise durch Isomerisierung von 3-Pentennitril (z.B. in Gegenwart von Ni(O)-Phosphatkomplexen und Lewissäuren, siehe Tolman et al. in Adv. in Catal., Vol. 33, 1985, S. 23; oder in Gegenwart von Hydroformylierungs-Katalysatoren unter Reaktionsbedingungen) zugänglich.

Die Umsetzung ("Hydroformylierung") des Pentennitrils oder Pentennitril-Gemisches erfolgt erfindungsgemäß mit Kohlenmonoxid und Wasserstoff in Gegenwart von Katalysatoren, die mindestens ein Element der achten Nebengruppe als aktive Komponente enthalten unter Erhalt eines Hydroformylierungsaustrages I.

Die Hydroformylierung wird üblicherweise bei Temperaturen im Bereich von 30 bis 180°C, insbesondere von 50 bis 130°C und Drücken im Bereich von 0,01 bar bis 100 bar, insbesondere 5 bar bis 20 bar, durchgeführt. Das Molverhältnis des verwendeten Gemischs aus Kohlenmonoxid und Wasserstoff wählt man im allgemeinen im Bereich von 1:100 bis 10:1, insbesondere von 1:20, bis 1:2.

In einer bevorzugten Ausführungsform führt man die Hydroformylierung in Gegenwart von unter den Reaktionsbedingungen inerten Lösungsmitteln wie aromatischen oder cycloaliphatischen Verbindungen durch wie Toluol oder Cyclohexan . Ebenfalls geeignet sind hochsiedende Ester wie Bis-(2-ethylhexyl)phthalat oder TEXANOL® (ein 2,2,4-Trimethylpentan-1,3-diolmonoisobutyrat der Firma EASTMAN). Besonders bevorzugt sind dabei diejenigen Formylvaleronitrile, die bei der Hydroformylierung der eingesetzten Pentennitrile entstehen sowie solche relativ zu den gebildeten Formylvaleronitrilen höhersiedenden Verbindungen, die durch Kondensation der gerade beschriebenen Formylvaleronitrile während der Hydroformylierung gebildet werden.

Als Katalysatoren setzt man erfindungsgemäß Katalysatoren ein, die mindestens ein Element der achten Nebengruppe wie Eisen, Cobalt, Nickel, Ruthenium, Rhodium, Palladium, Osmium, Iridium und Platin, als aktive Komponenten enthalten. Besonders bevorzugte Elemente sind Cobalt, Rhodium und Platin.

In einer besonders bevorzugten Ausführungsform setzt man Cokatalysatoren wie lipophile oder hydrophile Liganden zu, insbesondere die Sulfon-, Ammonium oder Carbonsäuresalze von Phosphinen, Diphosphinen, Phosphiten oder Di- sowie Polyphosphiten.

Besonders bevorzugt als Katalysatoren für die Hydroformylierung sind nach bisherigen Beobachtungen Rhodiumcarbonylkomplexe wie Rhodiumbis(carbonyl)acetylacetonat (Rh(CO)₂acac) in Verbindung mit Cokatalysatoren, ausgewählt aus der Gruppe, bestehend aus Triarylphosphinen wie Triphenylphosphin, Bis(diarylphosphino)alkanen wie 2,2'-Bis-diphenylphosphinomethyl-biphenyl oder Diphenylphosphinoethan, Triarylphosphiten wie Triphenylphosphit oder Tris-2-tert.-Butylphenylphosphit und Polyphosphiten der Formeln I und II, die beispielsweise aus EP-A 472 071, US-A 4,769,498, EP-A 149 894, EP-A 96 988 und EP-A 96 986 bekannt sind.

Polyphosphite der Formel I sind 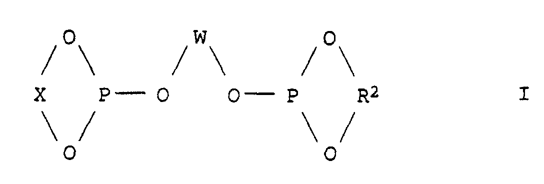 in der
- X: einen zweiwertigen Bisarylenrest oder R¹,
- W: einen zweiwertigen substituierten oder nicht substituierten Arylen-, Bisarylen- oder Alkylenrest und
- R¹ und R²: gleich oder verschieden sind und einen substituierten oder unsubstituierten Alkylen- oder ortho-Arylenrest bedeuten,

Von den Verbindungen der Formel I sind solche bevorzugt, bei denen die Reste X und W in der Formel I Bisarylenreste, insbesondere den Rest der Formel IV 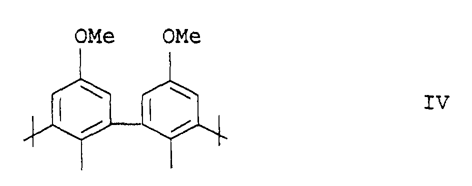 und R² einen ortho-Phenylen-, 2,2-dimethylpropylen-1,3- oder 1,1,2,2-Tetramethylethylenrest bedeuten. Ferner sind solche Verbindungen der Formel I bevorzugt, bei denen W, R¹ und R² unabhängig voneinander ortho-Phenylen-, 2,2-Dimethylpropylen-1,3- oder 1,1,2,2-Tetramethylethylenreste bedeuten.

Die Polyphosphite der Formel I können nach an sich bekannten Methoden durch eine geeignet gewählte Abfolge von Phosphorhalogenid-Alkohol-Kondensationsreaktion hergestellt werden.
a) Beispielsweise setzt man Phosphortrichlorid mit einem Diol unter Bildung eines Monochlorphosphites um:
b) setzt dieses Zwischenprodukt mit einem weiteren Diol unter Bildung des korrespondierenden hydroxylsubstituierten Diorganophosphitzwischenprodukt um:
c) läßt dieses Diorganophosphitzwischenprodukt mit Phosphortrichlorid unter Bildung des korrespondierenden Phosphordichloridzwischenproduktes reagieren;
d) und schließt mit der Reaktion dieses Dichlorids mit einem entsprechenden Diol unter Bildung des gewünschten Bisphosphites ab.

Während dieser Syntheseweg für die Herstellung von unsymmetrisch substituierten Phosphiten erforderlich ist, lassen sich symmetrisch substituierte Verbindungen durch Reaktion des Produktes von Schritt a) mit einem entsprechenden Diol im Molverhältnis 2:1 herstellen.

Die genannten Kondensationsreaktionen werden in der Regel in einem geeigneten Lösungsmittel, z.B. Toluol in Gegenwart einer Hilfsbase, wie Triethylamin, als HCl-Akzeptor durchgeführt.

Geeignete Verbindungen der Formel I sind z.B.

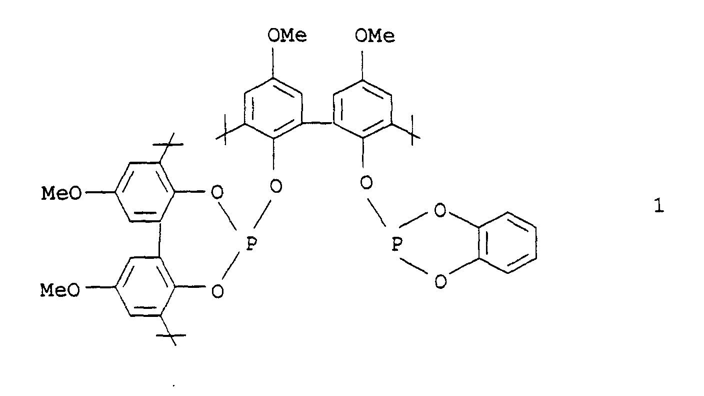

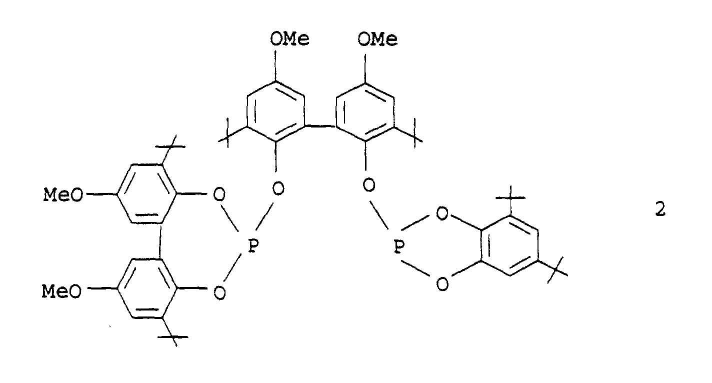

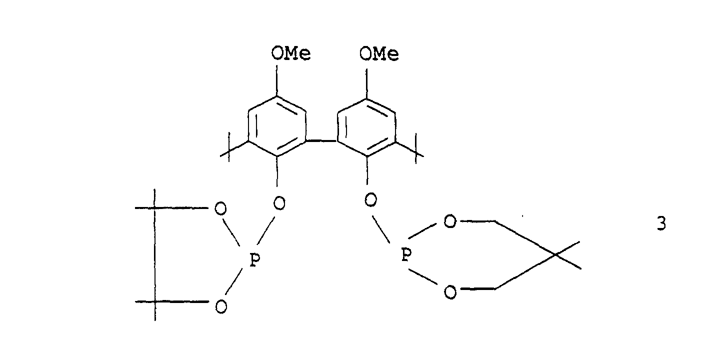

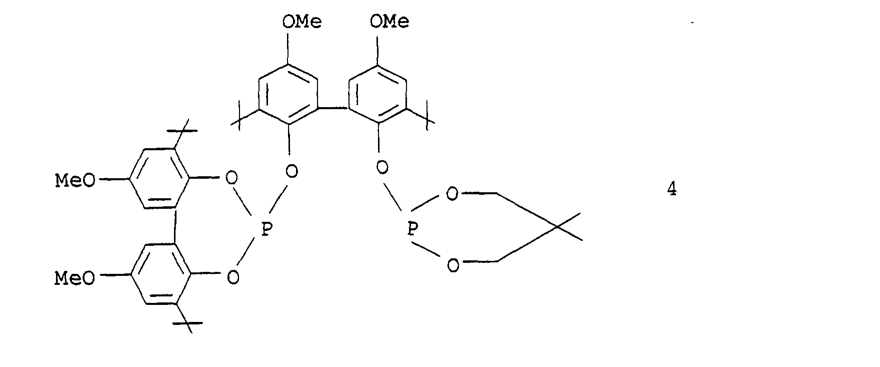

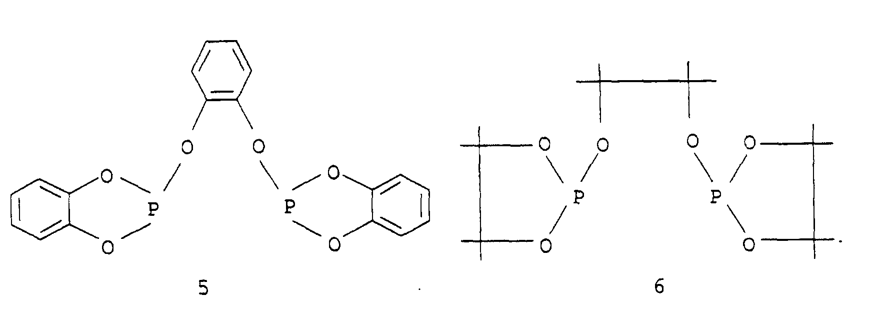

Polyphosphite der Formel II sind

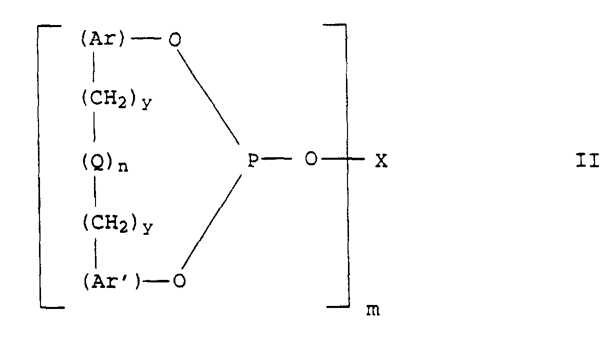

In der Formel II bedeuten
- Ar, Ar': jeweils ein Arylenradikal mit 6 bis 18 Kohlenstoffatomen, die gleich oder verschieden sein sowie substituiert oder nicht substituiert sein können;
- X: ein m-bindiges Radikal mit 2 bis 30 Kohlenstoffatomen, ausgewählt aus der Gruppe, bestehend aus Alkylen, Alkylen-oxy-alkylen, Arylen und einen Rest der Formel Arylen-(CH₂)_{y}-(Q)ₙ-(CH₂)_{y}-Arylen
in der die Arylenradikale wie oben definiert sind und
- y: 0 oder 1 ist;
- Q: eine bivalente Brückengruppe, ausgewählt aus der Gruppe, bestehend aus Sauerstoff, Schwefel, -CO- und -CR³R⁴-, bedeutet, in der R³ und R⁴ jeweils ein Wasserstoffatom oder ein Alkylrest mit 1 bis 12 Kohlenstoffatomen oder einen Phenyl-, Tolyl- oder Anisylrest bezeichnen und -NR⁵-, in der R⁵ ein Wasserstoffatom oder einen Methylrest bezeichnet;
- n: 0 oder 1 bedeutet und
- m: 2,3,4,5 oder 6 bezeichnet,

In bevorzugten Verbindungen der Formel II bezeichnen Ar und Ar' jeweils einen Phenylenrest, y und n O und m 2, wobei die beiden Phenylenreste in o-Stellung miteinander verbunden sind und in o- und p-Stellung zur Bindung an die Sauerstoffbrücke zum Phosphoratom Alkylgruppen mit 1 bis 4 Kohlenstoffatomen oder C₁-C₄-Alkoxygruppen, insbesondere Methoxy- und t-Butylgruppen, als Substituenten haben können.

Besonders bevorzugt ist das Phosphit der Formel III

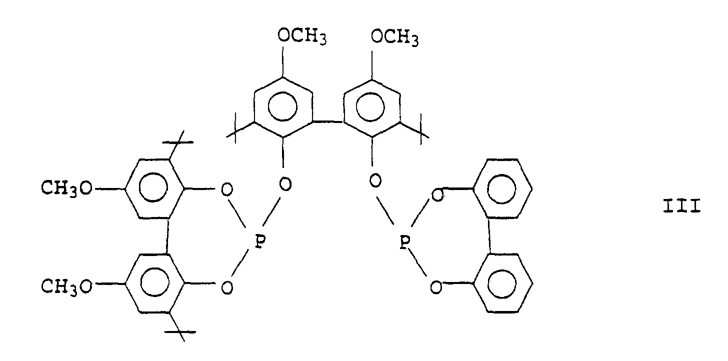

Das Molverhältnis von Pentennitril zu Katalysator wählt man im allgemeinen im Bereich von 100:1 bis 100.000:1, bevorzugt von 500:1 bis 10.000:1.

Das Molverhältnis von Katalysator zu Cokatalysator zu Pentennitrilen beträgt üblicherweise 1:1:100 bis 1:200:100000, insbesondere 1:2:500 bis 1:100:10000.

Aus dem Hydroformylierungsaustrag trennt man gewünschtenfalls Wasserstoff, Kohlenmonoxid, Katalysator und gegebenenfalls Lösungsmittel, falls man die Hydroformylierung in Gegenwart eines Lösungsmittels durchführte, ab unter Erhalt eines Hydroformylierungsaustrages II.

In einer besonders bevorzugten Ausführungsform führt man die abgetrennten Stoffe - Wasserstoff, Kohlenmonoxid, Katalysator und gegebenenfalls Lösungsmittel - in die Hydroformylierungsstufe a) zurück.

Aus dem Hydroformylierungsaustrag I oder II trennt man erfindungsgemäß 5-Formylvaleronitril ab. Bevorzugt führt man die Abtrennung durch Destillation durch.

In einer besonders bevorzugten Ausführungsform trennt man aus den Hydroformylierungsausträgen I oder II zunächst höhersiedende Bestandteile ("Hochsieder") und den Katalysator ab. Hierbei setzt man besonders bevorzugt thermisch schonende Verfahren ein, die durch beispielsweise sogenannte "Flashverdampfer", Fallfilmverdampfer oder Wischblattverdampfer in an sich bekannter Weise verwirklicht werden können. Die abgetrennten Hochsieder und den Katalysator, insbesondere Rh-haltige Katalysatoren, kann man gewünschtenfalls in die Hydroformylierungsstufe (Stufe a) zurückführen.

Im Falle der Verwendung Cobalt-haltiger Katalysatoren oxidiert man in einer bevorzugten Ausführungsform die Hydroformylierungsausträge I oder II mit Sauerstoff, bevorzugt mit Luft, um Cobalt in eine nichtflüchtige Form, insbesondere Co(II)-Salze, zu überführen.

Das auf diese Weise von Hochsiedern, beispielsweise Aldolkondensationsprodukte, und Katalysator befreite Gemisch unterwirft man bevorzugt einer weiteren Destillation, in der im Vergleich zu Formylvaleronitrilen leichtsiedende Anteile wie unumgesetzte Pentennitrile abgetrennt werden. Die so gewonnenen Pentennitrile kann man teilweise oder vollständig in die Hydroformylierungsstufe zurückführen.

Um das gewünschte Wertprodukt 5-Formylvaleronitril zu erhalten, wird bevorzugt das nach Abtrennung von Pentennitrilen gewonnene, überwiegend 5-, 4- und 3-Formylvaleronitril enthaltende Reaktionsgemisch fraktionierend destilliert. Dabei wird 5-Formylvaleronitril (Kp 95°C/3 mbar) von einem Gemisch aus 4- und 3-Formylvaleronitril (Kp 77-81°C/3 mbar) abgetrennt. Das Gemisch der verzweigten Formylvaleronitrile wird im allgemeinen aus dem Verfahren ausgeschleust.

Es war überraschend, daß die Siedepunktsdifferenz zwischen 5-Formylvaleronitril und den verzweigten Isomeren 4- und 3-Formylvaleronitril selbst bei niedrigen Vakua ausreichend groß ist. So beträgt die Siedepunktdifferenz beispielsweise bei einem Druck von 3 mbar 14°C. Somit ist auch im technisch relevanten Vakuumbereich, d.h. bei Drücken von nicht kleiner als 10 mbar, eine wirtschaftlich vertretbare Trennung möglich.

Es ist selbstredend, daß auch eine andere Gewinnung von 5-Formylvaleronitril möglich ist, beispielsweise, indem man die Destillation in nur einer Stufe oder in zwei Stufen durchführt.

Nach bisherigen Beobachtungen entsteht je nach Reaktionsbedingungen auch mehr oder weniger 6-Hydroxyvaleronitril. Dieses Produkt stellt insofern auch ein Wertprodukt dar, da es zusammen oder getrennt mit 5-Formylvaleronitril zu 6-Aminocapronitril umgesetzt werden kann.

Falls man Formylvaleronitrile als Lösungsmittel einsetzen möchte, so schleust man zweckmäßig den gewünschten Anteil an entstandenen Formylvaleronitrilen aus und führt den Rest im Kreis zurück.

Erfindungsgemäß setzt man 5-Formylvaleronitril bei Temperaturen im Bereich von 40°C bis 150°C, vorteilhaft von 50°C bis 140°C, insbesondere von 60°C bis 130°C, und Drücken im Bereich von 2 bis 350, vorteilhaft von 20 bis 300 bar, insbesondere von 40 bis 250 bar, mit Ammoniak und Wasserstoff in Gegenwart von Hydrierkatalysatoren in einem ersten Schritt (Stufe a)) unter Erhalt eines Hydrieraustrages um.

Die Umsetzung führt man bevorzugt in flüssigem Ammoniak als Lösungsmittel durch, wobei der Ammoniak gleichzeitig als Reaktionspartner dient. Die Ammoniak-Menge beträgt in der Regel 1 bis 80 Mol, insbesondere 10 bis 50 Mol Ammoniak pro Mol 5-Formylvaleronitril. Es kann auch vorteilhaft sein, zusätzlich zu Ammoniak ein unter den Reaktionsbedingungen inertes Lösungsmittel, wie z. B. Alkohole, Ester, Ether, Kohlenwasserstoffe mitzuverwenden, wobei man im allgemeinen ein Gewichtsverhältnis von Lösungsmittel zu 5-Formylvaleronitril im Bereich von 0,1 : 1 bis 5 : 1, vorzugsweise von 0,5 :1 bis 3 : 1, wählt. Besonders bevorzugt sind Alkohole wie Methanol und Ethanol.

Die Menge an Wasserstoff wählt man üblicherweise so, daß das Molverhältnis von Wasserstoff zu 5-Formylvaleronitril im Bereich von 1 : 1 bis 100 . 1, insbesondere von 5 : 1 bis 50 : 1 liegt. Als Katalysatoren setzt man erfindungsgemäß Hydrierkatalysatoren ein, die ausgewählt sind aus der Gruppe, bestehend aus Metallen oder Metallverbindungen von Rhenium, Kupfer und den Elementen der achten Nebengruppe (nachfolgend als "Hydriermetalle" bezeichnet), insbesondere Eisen, Cobalt, Nickel, Ruthenium, Rhodium, Palladium, Osmium, Iridium und Platin, besonders bevorzugt Ruthenium, Cobalt, Palladium und Nickel, mit der Maßgabe, daß der Hydrierkatalysator nicht Kupfer, Nickel oder Kupfer und Nickel als einzige Komponenten enthält.

Bei den erfindungsgemäß einsetzbaren Katalysatoren kann es sich um Voll- oder Trägerkatalysatoren handeln. Als Trägermaterialien kommen beispielsweise poröse Oxide wie Aluminiumoxid, Siliciumdioxid, Alumosilikate, Lanthanoxid, Titandioxid, Zirkondioxid, Magnesiumoxid, Zinkoxid und Zeolithe sowie Aktivkohle oder Mischungen der genannten Verbindungen in Betracht.

Die Katalysatoren können als Festbettkatalysatoren in Sumpf- oder Rieselfahrweise oder als Suspensionskatalysatoren eingesetzt werden. Dabei wählt man bevorzugt eine Katalysatorbelastung im Bereich von 0,1 bis 2,0, vorzugsweise von 0,3 bis 1 kg 5-Formylvaleronitril/l Katalysator • Stunde.

Es ist auch möglich, Verbindungen der oben genannten Metalle als homogen gelöste Hydrierkatalysatoren zu verwenden.

In einer bevorzugten Ausführungsform können die oben genannten Katalysatoren weiterhin 0,01 bis 25, vorzugsweise 0,1 bis 5 Gew.-%, bezogen auf die Gesamtmenge an Hydriermetallen (berechnet als Elemente) , mindestens eines Promotors auf der Basis eines Metalls, ausgewählt aus der Gruppe Kupfer, Silber, Gold, Mangan, Zink, Cadmium, Blei, Zinn, Scandium, Yttrium, Lanthan und der Lanthanidelemente, Titan, Zirkon, Hafnium, Chrom, Molybdän, Wolfram, Vanadium, Tantal, Antimon, Bismuth, Aluminium enthalten, sowie ferner durch 0,01 bis 5, vorzugsweise 0,1 bis 3 Gew.-%, bezogen auf die Hydriermetalle (berechnet als Elemente), einer Verbindung auf der Basis eines Alkalimetalles oder eines Erdalkalimetalles dotiert sein, vorzugsweise Alkalimetall- und Erdalkalihydroxide wie Lithiumhydroxid, Natriumhydroxid, Kaliumhydroxid, Rubidiumhydroxid, Cäsiumhydroxid, besonders bevorzugt Lithiumhydroxid.

Die im erfindungsgemäßen Verfahren eingesetzten Katalysatoren können z. B. sogenannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, z. B. schwerlösliche Hydroxide, Oxidhydrate, basische Salze oder Carbonate ausfällt, die erhaltenen Niederschläge anschließend trocknet und diese dann durch Calcinierung bei Temperaturen im Bereich von im allgemeinen 300 bis 700°C, insbesondere von 400 bis 600°C in die entsprechenden Oxide, Mischoxide und/oder gemischtvalentigen Oxide umwandelt, welche in der Regel durch eine Behandlung mit Wasserstoff oder mit Wasserstoff enthaltenden Gasen bei üblicherweise 50 bis 700°C, insbesondere 100 bis 400°C, zu den betreffenden Metallen und/oder oxidischen Verbindungen niederer Oxidationsstufe reduziert, und dabei in die eigentliche, katalytisch aktive Form überführt werden. Hierbei wird in der Regel so lange reduziert, bis kein Wasser mehr gebildet wird.

Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden. Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den oben genannten Fällungskatalysatoren, welche außer den katalytisch aktiven Komponenten noch zusätzlich ein Trägermaterial enthalten, eignen sich für das erfindungsgemäße Verfahren im allgemeinen solche Trägermaterialien, bei denen die katalytisch-hydrierend wirkende Komponenten, z. B. durch Imprägnierung, auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diesen Trägermaterialien z.B. durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden.

Eine andere Möglichkeit zur Aufbringung der katalytisch aktiven Metalle auf diese Träger besteht darin, die Träger mit Lösungen thermisch leicht zersetzbarer Salze, z. B. mit Nitraten oder thermisch leicht zersetzbaren Komplexverbindungen, z. B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen im Bereich von in der Regel 300 bis 600°C zu erhitzen. Diese thermische Zersetzung wird vorzugsweise unter einer Schutzgasatmosphäre vorgenommen. Geeignete Schutzgase können beispielsweise Stickstoff, Kohlendioxid, Wasserstoff oder ein Edelgas sein.

Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, daß höhere Gehalte dieser Trägerkatalysatoren an katalytisch aktiven Metallen üblicherweise zu höheren Raum-Zeit-Umsätzen führen können als niedrigere Gehalte. Im allgemeinen werden Trägerkatalysatoren verwendet, deren Gehalt an katalytisch aktiven Metallen 0,1 bis 90 Gew.-%, vorzugsweise 0,5 bis 40 Gew.-%, bezogen auf den gesamten Katalysator beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben nach bisherigen Beobachtungen aber auch unter- oder überschritten werden, ohne daß sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-A 2 519 817, EP-A 1 477 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z. B. durch Tränkung mit einem Salz oder Komplex der zuvor genannten Metalle erzeugt werden können.

Sowohl die Aktivierung der Fällungskatalysatoren als auch der Trägerkatalysatoren kann auch in situ zu Beginn der Reaktion durch den anwesenden Wasserstoff erfolgen, bevorzugt werden diese Katalysatoren jedoch vor ihrer Verwendung separat aktiviert.

Aus dem in Stufe a) des erfindungsgemäßen Verfahrens erhaltenen Hydrieraustrag gewinnt man nach üblichen Methoden wie Destillation 6-Aminocapronitril, gegebenenfalls zusammen mit Hexamethylendiamin (Stufe b)).

In einer bevorzugten Ausführungsform trennt man vor der Gewinnung von 6-Aminocapronitril und gewünschtenfalls Hexamethylendiamin in Stufe e) überschüssigen Ammoniak, Wasserstoff und gewünschtenfalls den Katalysator ab.

In einer weiteren bevorzugten Ausführungsform behandelt man 5-Formylvaleronitril zunächst bei Temperaturen im Bereich 40 bis 150°C mit Ammoniak (Stufe d') unter Erhalt eines ammoniakalischen Austrags. Dies kann beispielsweise in einem Vorreaktor erfolgen. Diese Umsetzung kann in Abwesenheit oder aber bevorzugt in Anwesenheit eines sauren, homogenen oder heterogenen Katalysators erfolgen. Die Katalysator-Belastung beträgt dabei (im Falle von heterogenen Katalysatoren) üblicherweise 0,1 bis 2,0 kg 5-Formylvaleronitril/l Katalysator • Stunde.

Der ammoniakalische Austrag kann dann gewünschtenfalls vom sauren Katalysator befreit werden (Stufe e').

In einer weiteren Stufe (Stufe f) setzt man den ammoniakalischen Austrag aus d') bzw. die ammoniakalische Lösung mit Ammoniak und Wasserstoff aus e') in Gegenwart von Hydrierkatalysatoren, ausgewählt aus der Gruppe, bestehend aus Metallen oder Metallverbindungen der Elemente Kupfer, Rhenium und Elementen der achten Nebengruppe, um unter Erhalt eines Hydrieraustrags, wobei das Verfahren in der Regel wie das weiter oben beschriebene Verfahren durchgeführt wird.

Im Anschluß daran (Stufe g)) gewinnt man in an sich bekannter Art und Weise 6-Aminocapronitril und gegebenenfalls Hexamethylendiamin aus dem Hydrieraustrag.

Als saure Katalysatoren kann man beispielsweise Zeolithe in der H-Form, saure Ionentauscher, Heteropolysäuren, saure und supersaure Metalloxide, die gegebenenfalls mit Sulfat oder Phosphat dotiert wurden und anorganische oder organische Säuren, verwenden.

Als Zeolithe eigenen sich beispielsweise Vertreter aus der Mordenit-Gruppe oder engporige Zeolithe vom Erionit- bzw. Chabasit-Typ oder Zeolithe von Faujasit-Typ, z. B. Y-, X- oder L-Zeolithe. In diese Gruppe gehören auch die sogenannten "ultrastabilen" Zeolithe des Faujasittyps, d. h. dealuminierte Zeolithe.

Besonders vorteilhaft unter den Zeolithen sind solche mit Pentasilstruktur wie ZSM-5, ZSM-11 und ZBM-10. Diese haben als Grundbaustein einen aus SiO₂-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.

Die erfindungsgemäß eingesetzten Heteropolysäuren sind anorganische Polysäuren, die im Gegensatz zu Isopoylsäuren mindestens zwei verschiedene Zentralatome besitzen. Als Beispiele seien Dodecawolframphosphorsäure H₃PW₁₂O₄₀ • xH₂O, Dodecamolybdophosphorsäure H₃PMo₁₂O₄₀ • xH₂O genannt. Prinzipiell können die in EP-A 158 229 genannten Katalysatoren und Katalysatorkombinationen eingesetzt werden.

Bevorzugte Heteropolysäuren sind Heteropolysäuren von Molybdän oder Wolfram mit Phosphorsäure, Tellursäure, Selensäure, Arsensäure, Kieselsäure, insbesondere mit Phosphorsäure.

Die Protonen der Heteropolysäuren können teilweise durch Metallionen ersetzt sein, bevorzugt sind dabei die Alkali- und Erdalkalimetallionen.

Bevorzugte saure Ionentauscher sind z. B. vernetzte Polystyrole mit Sulfonsäuregruppen.

Saure Metalloxide sind beispielsweise SiO₂, Al₂O₃, ZrO₂, Ga₂O₃, PbO₂, Sc₂O₃, La₂O₃, TiO₂, SnO₂ usw. oder Kombinationen einzelner Oxide. Die Oxide können zur Säurestärkeerhöhung auch mit Mineralsäuren wie z. B. Schwefelsäure behandelt werden.

Als Säuren sind beispielsweise Mineralsäuren wie Schwefelsäure und Phosphorsäure sowie organische Säuren, wie z. B. Sulfonsäuren geeignet.

Als supersaure Metalloxide sind z. B. Sulfat-dotiertes ZrO₂ oder Molybdän- oder Wolfram enthaltendes ZrO₂ geeignet.

In einer weiteren bevorzugten Ausführungsform führt man die Hydrierung an einem Hydriermetall, aufgebracht auf einem der genannten oxidischen Träger, durch. Nach Entfernung von überschüssigem Wasserstoff und gegebenenfalls des Katalystors wird der Hydrieraustrag bevorzugt durch fraktionierende Destillation zu 6-Aminocapronitril und gegebenenfalls Hexamethylendiamin aufgearbeitet.

Nach dem erfindungsgemäßen Verfahren erhält man 6-Aminocapronitril mit sehr guten Umsätzen, guten Ausbeuten und Selektivitäten. Es ist auch möglich, durch Variation von Temperatur und Katalysatorbelastung Gemische aus 6-Aminocapronitril und Hexamethylendiamin zu erhalten. Dabei begünstigen höhere Temperaturen und niedrige Katalysatorbelastungen die Bildung von Hexamethylendiamin, niedrigere Temperaturen und hohe Katalysatorbelastungen die Bildung von 6-Aminocapronitril.

6-Aminocapronitril und Hexamethylendiamin stellen wichtige Faservorprodukte dar. 6-Amino-capronitril läßt sich zu Caprolactam, dem Vorprodukt für die Herstellung von Nylon 6, cyclisieren. Hexamethylendiamin wird hauptsächlich mit Adipinsäure zu dem sogenannten AH-Salz, der Vorstufe von Nylon 6.6, umgesetzt.

### Beispiele

### Beispiel 1

In einem 1 1-Hubrührautoklaven wurden 100 g 3-Pentennitril, 350 g m-Xylol als Lösungsmittel und 0,08 Gew.-% Cobalt in Form von Dicobaltoctacarbonyl als Katalysator auf 105°C aufgeheizt. Nach Erreichen der Endtemperatur wurde das Reaktionsgemisch mit einem Gemisch aus 50 Vol.-% CO und 50 Vol.-% H₂ und einem Druck von 140 bar versetzt. Während der Reaktion wurde der Druck im Reaktor durch Nachpressen eines Gasgemisches aus 50 Vol.-% CO und 50 Vol.-% H₂ konstant gehalten. Nach einer Reaktionszeit von 3 Stunden wurde die Reaktion durch Abkühlen und Druckablassen abgebrochen. Eine GC-Analyse des Reaktionsgemisches ergab folgende Zusammensetzung des Reaktionsgemisches (in Mol-%):

| | |
|---|---|
| Umsatz | 7,5 (Umsatz an 3-Pentennitril) |
| Selektivität | |
| Valeronitril | 9,0 |
| 5-Formylvaleronitril | 39,0 |
| 3- und 4-Formylvaleronitril | 52,0 |

Die Ausbeute an Formylvaleronitrilen (Summe aus 3-, 4- und 5-FVN) betrug 6,8 %, das Molverhältnis von 5-Formylvaleronitril zu 3- und 4-Formylvaleronitril betrug 43 : 57.

### Beispiel 2

In einem 1 1-Hubrührautoklaven wurden 100 g 3-Pentennitril, 350 g m-Xylol als Lösungsmittel und 0,04 Gew.-% Cobalt in Form von Dicobaltoctacarbonyl als Katalysator auf 170°C aufgeheizt. Nach Erreichen der Endtemperatur wurde das Reaktionsgemisch mit einem Gemisch aus 50 Vol.-% CO und 50 Vol.-% H₂ und einem Druck von 280 bar versetzt. Während der Reaktion wurde der Druck im Reaktor durch Nachpressen eines Gasgemisches aus 50 Vol.-% CO und 50 Vol.-% H₂ konstant gehalten. Nach einer Reaktionszeit von 2 Stunden wurde die Reaktion durch Abkühlen und Druckablassen abgebrochen. Eine GC-Analyse des Reaktionsgemisches ergab folgende Zusammensetzung des Reaktionsgemisches (in Mol-%) :

| | |
|---|---|
| Umsatz | > 99,9 |
| Selektivität | |
| Valeronitril | 41,0 |
| 5-Formylvaleronitirl | 16,0 |
| 3- und 4-Formylvaleronitril | 6,4 |
| 6-Hydroxycapronitril | 23,0 |
| 4- und 5-Hydroxymethylvaleronitril | 3,6 |

Die Ausbeute an 5-Formylvaleronitril und 6-Hydroxycapronitril betrug zusammen 39 % und das Molverhältnis 5-Formylvaleronitril zu 3- und 4-Formylvaleronitril betrug 60 : 40.

### Beispiel 3

In einem 300 ml-HC-Autoklaven mit Magnetrührung wurden 20 g 3-Pentennitril, 100 g Toluol als Lösungsmittel, 100 ppm Rhodium in Form der Komplexverbindung Rh(CO)₂acac (acac = Acetylacetonat, 25 mg) und das Bisphosphit der Formel III (557 mg) (sechsfacher molaren Überschuß bezogen auf Rhodium) 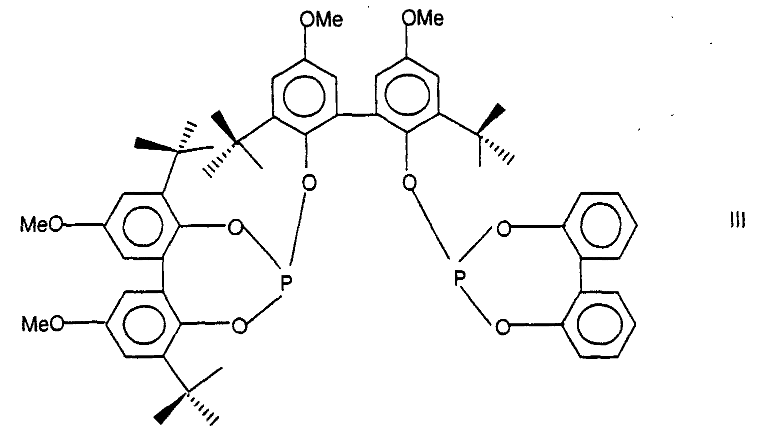 auf 100 °C aufgeheizt. Nach Erreichen der Endtemperatur wurde das Reaktionsgemisch mit mit einem Gemisch aus 50 Vol.-% CO und 50 Vol.-% H₂ bei einem Druck von 5 bar versetzt. Während der Reaktion wurde der Druck im Reaktor durch Nachpressen eines Gasgemisches aus 50 Vol.-% CO und 50 Vol.-% H₂ über einen Druckregler auf 5 bar konstant gehalten. Nach einer Reaktionszeit von 5 Stunden wurde die Reaktion durch Abkühlen auf Raumtemperatur und Druckablassen abgebrochen. Eine GC-Analyse des Reaktionsgemisches ergab folgende Resultate (Angaben in Mol-%):

| | |
|---|---|
| Umsatz: | > 99,9 |
| Selektivität | |
| 2-, 4-Pentennitril | 19,5 |
| Valeronitril | 21,3 |
| 5-Formylvaleronitril | 29,7 |
| 3- und 4-Formylvaleronitril | 29,5 |

Die Ausbeute an Wertprodukt 5-Formylvaleronitril betrug 29,7 %, das Molverhältnis von 5-Formylvaleronitril zu 3- und 4-Formylvaleronitril betrug 50,2:49,8.

### Beispiel 4

In einem 300 ml-HC-Autoklaven mit Magnetrührung wurden 20 g 3-Pentennitril, 100 g Palatinol®C (Di-n-Butylphthalat) als Lösungsmittel, 100 ppm Rhodium in Form der Komplexverbindung Rh(CO)₂acac (acac = Acetylacetonat, 25 mg) und Triphenylphosphit in einem auf Rhodium bezogenen fünfundzwanzigfachen molaren Überschuß auf 100 °C aufgeheizt. Nach Erreichen der Endtemperatur wurde das Reaktionsgemisch mit mit einem Gemisch aus 50 Vol.-% CO und 50 Vol.-% H₂ bei einem Druck von 5 bar versetzt. Während der Reaktion wurde der Druck im Reaktor durch Nachpressen eines Gasgemisches aus 50 Vol.-% CO und 50 Vol.-% H₂ über einen Druckregler auf 5 bar konstant gehalten. Nach einer Reaktionszeit von 5 Stunden wurde die Reaktion durch Abkühlen auf Raumtemperatur und Druckablassen abgebrochen. Eine GC-Analyse des Reaktionsgemisches ergab folgende Resultate (Angaben in Mol-%):

| | |
|---|---|
| Umsatz: | 98,6 |
| Selektivität | |
| 2-, 4-Pentennitrile | 0 |
| Valeronitril | 10,3 |
| 5-Formylvaleronitril | 18,2 |
| 3- und 4-Formylvaleronitril | 71,5 |

Die Ausbeute an Wertprodukt 5-Formylvaleronitril betrug 17,9 %, das Molverhältnis 5-Formylvaleronitril zu 3- und 4-Formylvaleronitril betrug 20 : 80.

### Beispiel 5

In einem 300 ml-HC-Autoklaven mit Magnetrührung wurden 20 g 4-Pentennitril, 100 g Toluol als Lösungsmittel, 100 ppm Rhodium in Form der Komplexverbindung Rh(CO)₂acac (acac = Acetylacetonat, 25 mg) und das Bisphosphit der Formel III (557 mg) (sechsfacher molaren Überschuß bezogen auf Rhodium) auf 100 °C aufgeheizt. Nach Erreichen der Endtemperatur wurde das Reaktionsgemisch mit mit einem Gemisch aus 50 Vol.-% CO und 50 Vol.-% H₂ bei einem Druck von 5 bar versetzt. Während der Reaktion wurde der Druck im Reaktor durch Nachpressen eines Gasgemisches aus 50 Vol.-% CO und 50 Vol.-% H₂ über einen Druckregler auf 5 bar konstant gehalten. Nach einer Reaktionszeit von 5 Stunden wurde die Reaktion durch Abkühlen auf Raumtemperatur und Druckablassen abgebrochen. Eine GC-Analyse des Reaktionsgemisches ergab folgende Resultate (Angaben in Mol-%):

| | |
|---|---|
| Umsatz: | > 99,9 |
| Selektivität | |
| 2-, 4-Pentennitrile | 34,0 |
| Valeronitril | 6,0 |
| 5-Formylvaleronitril | 49,2 |
| 3- und 4-Formylvaleronitril | 10,8 |

Die Ausbeute an Wertprodukt 5-Formylvaleronitril betrug 49,2 %, das Molverhältnis von 5-Formylvaleronitril zu 3- und 4-Formylvaleronitril betrug 82 : 18.

### Beispiel 6

Mehrere nach Beispiel 3 erhaltene Hydroformylierungsausträge wurden vereinigt. Nach Abdestillieren von 2-, 4-Pentennitril und Valeronitril wurden 130 g eines Reaktionsgemisches erhalten, das laut gaschromatographischer Analyse zu 48 Gew.-% aus 5-Formylvaleronitril und zu 46 Gew.-% aus 4- und 3-Formylvaleronitril bestand. Durch fraktionierende Destillation an einer Drehbandkolonne wurden hieraus 59 g 5-Formylvaleronitril vom Siedepunkt 93 - 95 °C/3 mbar (Reinheit 99 %) und 56 g 4- + 3-Formylvaleronitril vom Siedepunkt 77 - 81 °C/3 mbar (Reinheit 4- + 3-Isomeres 96 %) erhalten.

### Beispiel 7

In einem 300 ml Autoklaven mit Probenahmeschleuse (Werkstoff HC 4) wurden 11 g 5-Formylvaleronitril und 3 g Ru (3 %)/Al₂O₃ (4 mm-Stränge) unter Schutzgas (Argon) eingefüllt. Anschließend wurde der Autoklav verschlossen und 150 ml NH₃ wurden aufgepreßt. Die Durchmischung erfolgte mit einem Magnetrührer. Nach Erhitzen auf 80 °C (Eigendruck: ca. 39 bar) wurde das Gemisch noch 2 Stunden bei 80 °C gehalten und dann der Gesamtdruck durch Wasserstoff auf 70 bar erhöht. Der Druck von 70 bar wurde durch ständiges Nachpressen von Wasserstoff gehalten. Nach 25 Stunden wurde der Autoklav entspannt und der Hydrieraustrag gaschromatographisch analysiert. Als Produkte entstanden 73 % 6-Aminocapronitril und 12 % Hexamethylendiamin. Der Umsatz betrug 100 %.

## Patentansprüche

1. Verfahren zur Herstellung von 6-Aminocapronitril oder 6-Aminocapronitril/Hexamethylendiamin-Gemischen, **dadurch gekennzeichnet, daß** man
a) mindestens ein Pentennitril, ausgewählt aus der Gruppe, bestehend aus 2-, 3- und 4- Pentennitril mit Kohlenmonoxid und Wasserstoff in Gegenwart von Katalysatoren umsetzt, die mindestens ein Element der achten Nebengruppe als aktive Komponente enthalten, unter Erhalt eines Hydroformylierungsaustrages I,
b) aus dem Hydroformylierungsaustrag I gewünschtenfalls Kohlenmonoxid, Wasserstoff und den Katalysator abtrennt unter Erhalt eines Hydroformylierungsaustrages II,
c) aus dem Hydroformylierungsaustrag I oder II 5-Formylvaleronitril abtrennt,
d) das abgetrennte 5-Formylvaleronitril mit Ammoniak und Wasserstoff in Gegenwart von Hydrierkatalysatoren, ausgewählt aus der Gruppe, bestehend aus Rhenium, Kupfer und deren Verbindungen sowie Metallen und Metallverbindungen der achten Nebengruppe, umsetzt unter Erhalt eines Hydrieraustrages, und
e) aus dem Hydrieraustrag 6-Aminocapronitril und gegebenenfalls Hexamethylendiamin gewinnt.

2. Verfahren nach Anspruch 1, **dadurch gekennzeichnet, daß** man vor der Gewinnung von 6-Aminocapronitril und gegebenenfalls Hexamethylendiamin in Stufe e) überschüssigen Ammoniak, Wasserstoff und gewünschtenfalls den Hydrierkatalysator abtrennt.

3. Verfahren nach den Ansprüchen 1 oder 2, **dadurch gekennzeichnet, daß** man in Stufe a) Katalysatoren verwendet, die Cobalt oder Rhodium oder Platin als aktive Komponente enthalten.

4. Verfahren nach den Ansprüchen 1 bis 2, **dadurch gekennzeichnet, daß** man die Umsetzung in Stufe a) in Gegenwart von Rhodiumcarbonylkomplexen als Katalysatoren durchführt, wobei man Cokatalysatoren, ausgewählt aus der Gruppe, bestehend aus Triarylphosphinen, Bis(diarylphosphino)alkanen, Triarylphosphiten oder Polyphosphiten der Formeln I und II 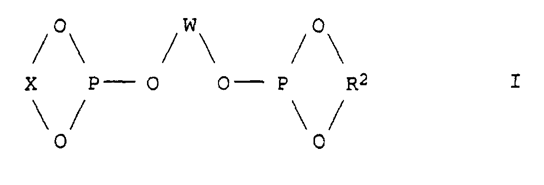 in der
X einen zweiwertigen Bisarylenrest oder R¹,
W einen zweiwertigen substituierten oder nicht substituierten Arylen-, Bisarylen- oder Alkylenrest und
R¹ und R² gleich oder verschieden sind und einen substituierten oder unsubstituierten Alkylen oder ortho-Arylenrest bedeuten, 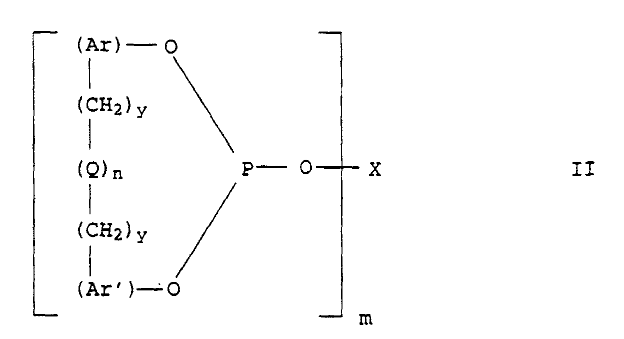
wobei
Ar, Ar' jeweils ein Arylenradikal mit 6 bis 18 Kohlenstoffatomen, die gleich oder verschieden sein sowie substituiert oder nicht substituiert sein können;
X ein m-bindiges Radikal mit 2 bis 30 Kohlenstoffatomen, ausgewählt aus der Gruppe, bestehend aus Alkylen, Alkylen-oxy-alkylen, Arylen und ein Rest der Formel
Arylen-(CH₂)_{y}-(Q)ₙ-(CH₂) -_{y}-Arylen
in der die Arylenradikale wie oben definiert sind und
y 0 oder 1 ist;
Q eine bivalente Brückengruppe, ausgewählt aus der Gruppe, bestehend aus Sauerstoff, Schwefel, -CO- und -CR³R⁴-, bedeutet, in der R³ und R⁴ jeweils ein Wasserstoffatom oder ein Alkylrest mit 1 bis 12 Kohlenstoffatomen oder einen Phenyl-, Tolyl- oder Anisylrest bezeichnen und -NR⁵-, in der R⁵ ein Wasserstoffatom oder einen Methylrest bezeichnet;
n 0 oder 1 bedeutet und
m eine ganze Zahl von 2 bis 6 bezeichnet,
zusetzt, wobei die Cokatalysatoren gewünschtenfalls mit Sulfonat- oder Carboxylatgruppen versetzt sein können.

5. Verfahren nach Anspruch 4, **dadurch gekennzeichnet, daß** man ein molares Verhältnis von Cokatalysator (gerechnet als Äquivalent Phosphor) zu Rhodium von 1 : 1 bis 300 : 1 wählt.

6. Verfahren nach den Ansprüchen 1 bis 5, **dadurch gekennzeichnet, daß** man die Umsetzung in Stufe a) und/oder d) in Gegenwart eines Lösungsmittel durchführt.

7. Verfahren zur Herstellung von 6-Aminocapronitril oder 6-Aminocapronitril/Hexamethylendiamin-Gemischen gemäß Anspruch 1, **dadurch gekennzeichnet, daß** man anstelle der Stufen d) und e) folgende Stufen durchführt:
d') 5-Formylvaleronitril zunächst mit Ammoniak, gewünschtenfalls in Gegenwart eines sauren Katalysators, behandelt unter Erhalt eines ammoniakalischen Austrags,
e') gewünschtenfalls anschließend den sauren Katalysator abtrennt unter Erhalt einer ammoniakalischen Lösung,
f) den ammoniakalischen Austrag aus d') bzw. die Lösung aus e') mit Ammoniak und Wasserstoff in Gegenwart von Hydrierkatalysatoren, ausgewählt aus der Gruppe, bestehend aus Kupfer, Rhenium und deren Verbindungen sowie Metallen und Metallverbindungen der achten Nebengruppe, umsetzt unter Erhalt eines Hydrieraustrages und
g) aus dem Hydrieraustrag 6-Aminocapronitril und gegebenenfalls Hexamethylendiamin gewinnt.

## Claims

1. A process for preparing 6-aminocapronitrile or 6-aminocapronitrile/hexamethylenediamine mixtures, which comprises
a) reacting at least one pentenenitrile selected from the group consisting of 2-pentenenitrile, 3-pentenenitrile and 4-pentenenitrile with carbon monoxide and hydrogen in the presence of a catalyst containing at least one element of subgroup eight as active component to obtain a hydroformylation effluent I,
b) optionally removing carbon monoxide, hydrogen and the catalyst from the hydroformylation effluent I to obtain a hydroformylation effluent II,
c) removing 5-formylvaleronitrile from said hydroformylation effluent I or II,
d) reacting the removed 5-formylvaleronitrile with ammonia and hydrogen in the presence of a hydrogenation catalyst selected from the group consisting of rhenium, copper and compounds thereof, and also metals and metal compounds of subgroup eight to obtain a hydrogenation effluent, and
e) isolating 6-aminocapronitrile with or without hexamethylenediamine from the hydrogenation effluent.

2. A process as claimed in claim 1, wherein excess ammonia, hydrogen and optionally the hydrogenation catalyst are removed prior to the isolation of 6-aminocapronitrile with or without hexamethylenediamine in step e).

3. A process as claimed in claim 1 or 2, wherein step a) utilizes a catalyst containing cobalt or rhodium or platinum as active component.

4. A process as claimed in claim 1 or 2, wherein the reaction of step a) is carried out in the presence of a rhodium carbonyl complex catalyst by adding a cocatalyst selected from the group consisting of triarylphosphines, bis(diarylphosphino)alkanes, triaryl phosphites and polyphosphites of the formulae I and II 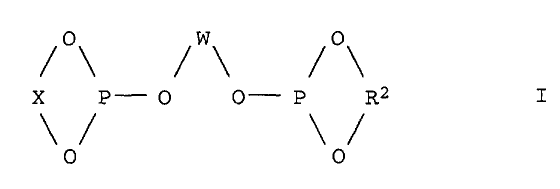 where
X is a bivalent bisarylene radical or R¹,
W is a bivalent substituted or unsubstituted arylene, bisarylene or alkylene radical, and
R¹ and R² are identical or different and each is a substituted or unsubstituted alkylene or ortho-arylene radical, 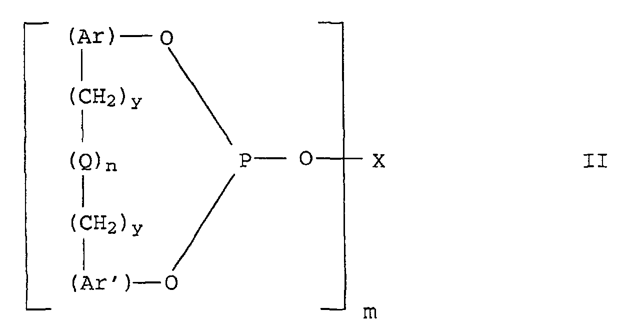
where
Ar and Ar' are identical or different, substituted or unsubstituted arylene radicals having from 6 to 18 carbon atoms;
X is an m-binding radical having from 2 to 30 carbon atoms and selected from the group consisting of alkylene, alkylene-oxy-alkylene, arylene and a radical of the formula
arylene- (CH₂)_{y}-(Q)ₙ- (CH₂)_{y}-arylene
where the arylene radicals are each as defined above and
y is 0 or 1;
Q is a bivalent bridging group selected from the group consisting of oxygen, sulfur, -CO-, -CR³R⁴-, where R³ and R⁴ are each hydrogen, alkyl having from 1 to 12 carbon atoms or a phenyl, tolyl or anisyl radical, and -NR⁵-, where R⁵ is hydrogen or methyl;
n is 0 or 1, and
m is an integer from 2 to 6,
and optionally containing sulfonate or carboxylate groups.

5. A process as claimed in claim 4, wherein the cocatalyst is added in a molar ratio of cocatalyst (calculated as equivalents of phosphorus) to rhodium within the range from 1:1 to 300:1.

6. A process as claimed in any of claims 1 to 5, wherein the reaction of step a) or d) is carried out in the presence of a solvent.

7. A process for the production of 6-aminocapronitrile or mixtures of 6-aminocapronitrile and hexamethylenediamine as claimed in claim 1, wherein steps d) and e) are replaced by the steps of:
d') treating 5-formylvaleronitrile first with ammonia, optionally in the presence of an acidic catalyst, to obtain an ammoniacal effluent,
e') then optionally removing the acidic catalyst to obtain an ammoniacal solution,
f) reacting the ammoniacal effluent of d'), or the solution of e'), with ammonia and hydrogen in the presence of a hydrogenation catalyst selected from the group consisting of copper, rhenium and compounds thereof and also metals and metal compounds of subgroup eight to obtain a hydrogenation effluent, and
g) isolating 6-aminocapronitrile with or without hexamethylenediamine from the hydrogenation effluent.

## Revendications

1. Procédé de préparation de 6-aminocapronitrile ou de mélanges de 6-aminocapronitrile/hexaméthylènediamine, **caractérisé en ce que**
a) l'on fait réagir au moins un nitrile penténique, choisi dans le groupe formé par les nitriles 2-, 3- et 4-penténiques, avec du monoxyde de carbone et de l'hydrogène, en présence de catalyseurs, contenant au moins un élément du huitième sous-groupe en tant que composant actif, avec obtention d'un produit d'hydroformylation I,
b) on sépare éventuellement du produit d'hydroformylation I le monoxyde de carbone, l'hydrogène et le catalyseur, avec obtention d'un produit d'hydroformylation II,
c) on sépare du produit d'hydroformylation I ou II du 5-formylvaléronitrile,
d) on fait réagir le 5-formylvaléronitrile isolé avec de l'ammoniac et de l'hydrogène en présence de catalyseurs d'hydrogénation, choisis dans le groupe formé par le rhénium, le cuivre et leurs composés, ainsi que des métaux et des composés métalliques du huitième sous-groupe, avec obtention d'un produit d'hydrogénation, et
e) on obtient à partir du produit d'hydrogénation du 6-aminocapronitrile et éventuellement de l'hexaméthylènediamine.

2. Procédé selon la revendication 1, **caractérisé en ce que** l'on sépare, avant l'obtention de 6-aminocapronitrile et éventuellement d'hexaméthylènediamine à l'étape e), l'ammoniac et l'hydrogène excédentaires, et éventuellement le catalyseur d'hydrogénation.

3. Procédé selon les revendications 1 ou 2, **caractérisé en ce que** l'on utilise à l'étape a) des catalyseurs contenant du cobalt ou du rhodium ou du platine en tant que composant actif.

4. Procédé selon les revendications 1 à 2, **caractérisé en ce que** l'on entreprend la réaction de l'étape a) en présence de complexes de rhodium carbonyle en tant que catalyseurs, et que l'on ajoute des cocatalyseurs choisis dans le groupe formé par des triarylphosphines, des bis(diarylphosphino)alcanes, des phosphites de triaryle ou des polyphosphites des formules I et II 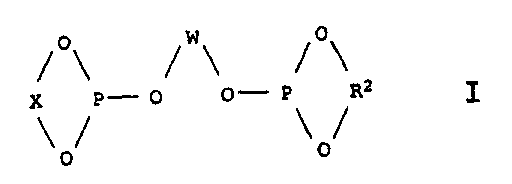 où
X représente un reste bisarylène bivalent ou R¹,
w représente un reste arylène, bisarylène ou alkylène bivalent substitué ou non substitué, et
R¹ et R² sont identiques ou différents et représentent un reste alkylène ou ortho-arylène substitué ou non substitué, 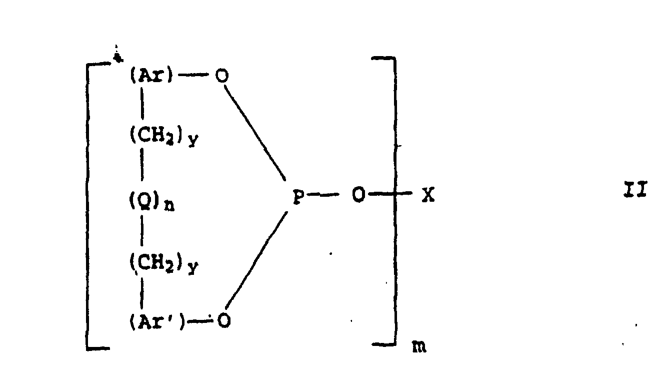
où
Ar, Ar' représentent à chaque fois un radical arylène comportant de 6 à 18 atomes de carbone, et peuvent être identiques ou différents ainsi que substitués ou non substitués,
X représente un radical à m valences comportant de 2 à 30 atomes de carbone, choisi dans le groupe formé par l'alkylène, l'alkylène-oxy-alkylène, l'arylène et un radical de la formule
arylène-(CH₂)_{y}-(Q)ₙ-(CH₂)-_{y}-arylène
dans laquelle les radicaux arylène sont tels que définis ci-avant et
y a une valeur de 0 ou 1;
Q représente un radical bivalent, choisi dans le groupe formé par l'oxygène, le soufre, -CO- et -CR³R⁴-, où R³ et R⁴ représentent à chaque fois un atome d'hydrogène ou un radical alkyle comportant de 1 à 12 atomes de carbone ou un radical phényle, tolyle ou anisyle, et -NR⁵-, où R⁵ représente un atome d'hydrogène ou un radical méthyle;
n a une valeur de 0 ou 1 et
m est un nombre entier de 2 à 6,
les cocatalyseurs pouvant éventuellement être mélangés à des radicaux sulfonate ou carboxylate.

5. Procédé selon la revendication 4, **caractérisé en ce que** l'on choisit une proportion molaire de cocatalyseur (calculé en équivalent de phosphore) au rhodium de 1:1 à 300:1.

6. Procédé selon les revendications 1 à 5, **caractérisé en ce que** l'on entreprend la réaction de l'étape a) et/ou de l'étape d) en présence d'un solvant.

7. Procédé de préparation de 6-aminocapronitrile ou de mélanges de 6-aminocapronitrile/hexaméthylènediamine selon la revendication 1, **caractérisé en ce qu'**au lieu des étapes d) et e), o, entreprend les étapes suivantes:
d') on traite d'abord le 5-formylvaléronitrile par de l'ammoniac, éventuellement en présence d'un catalyseur acide, avec obtention d'un produit ammoniacal,
e') on sépare éventuellement ensuite le catalyseur acide avec obtention d'une solution ammoniacale,
f) on fait réagir le produit ammoniacal de d') ou la solution de e') avec de l'ammoniac et de l'hydrogène en présence de catalyseurs d'hydrogénation choisis dans le groupe formé par le cuivre, le rhénium et leurs composés ainsi que des métaux et des composés métalliques du huitième sous-groupe, avec obtention d'un produit d'hydrogénation, et
g) on isole du produit d'hydrogénation du 6-aminocapronitrile et éventuellement de l'hexaméthylènediamine.
